(19) Europäisches Patentamt
European Patent Office
Office européen des brevets

(11) Veröffentlichungsnummer: **0 606 325 B1**

(12) # EUROPÄISCHE PATENTSCHRIFT

(45) Veröffentlichungstag der Patentschrift: **29.11.95**

(21) Anmeldenummer: **92920594.6**

(22) Anmeldetag: **25.09.92**

(86) Internationale Anmeldenummer: **PCT/EP92/02223**

(87) Internationale Veröffentlichungsnummer: **WO 93/05764 (01.04.93 93/09)**

(51) Int. Cl.[6]: **A61K 7/50**, A61K 7/48, A61K 7/08, C11D 1/825, A61K 31/77

(54) **REINIGUNGSFLÜSSIGKEIT, INSBESONDERE FÜR NOTFALLSETS, UND IHRE VERWENDUNG.**

(30) Priorität: **26.09.91 DE 4132129**
**02.10.91 DE 4132886**

(43) Veröffentlichungstag der Anmeldung: **20.07.94 Patentblatt 94/29**

(45) Bekanntmachung des Hinweises auf die Patenterteilung: **29.11.95 Patentblatt 95/48**

(84) Benannte Vertragsstaaten: **AT CH DE FR GB LI NL**

(56) Entgegenhaltungen:
**EP-A- 0 115 888**
**EP-A- 0 408 965**
**EP-A- 0 471 606**
**WO-A-91/09925**
**GB-A- 2 242 686**

(73) Patentinhaber: **BOUS, Klaus**
**P.O. Box 30 39**
**Sherwood Park,**
**Alberta T8A 2A6 (CA)**

(72) Erfinder: **BOUS, Klaus**
**P.O. Box 30 39**
**Sherwood Park,**
**Alberta T8A 2A6 (CA)**

(74) Vertreter: **Reinhard - Skuhra - Weise & Partner**
**Postfach 44 01 51**
**D-80750 München (DE)**

EP 0 606 325 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Reinigungsflüssigkeit, die insbesondere für den Einsatz in Notfallsets bestimmt ist, sowie ihre Verwendung als allgemeines Reinigungsmittel, als Mittel zum Reinigen und/oder Dekontaminieren von Haut bei Verletzungen und/oder Kontaminationen bei Mensch und Tier und zur medizinischen Hautbehandlung und Hautreinigung.

Reinigungsflüssigkeiten, auch für medizinische Zwecke, sind in großer Anzahl bekannt. Soweit sie im Haushalt oder für medizinische Zwecke zur Anwendung kommen, enthalten sie als Grundbestandteil in der Regel Wasser, dem verschiedenen waschaktive Substanzen zugesetzt sind. Für technische Zwecke werden regelmäßig noch nicht wäßrige Lösungsmittel als Reinigungsflüssigkeiten verwandt, wenn sich auch - aus Gründen des Umweltschutzes - wäßrige Reinigungsflüssigkeiten mehr und mehr durchsetzen.

Alle diese Reinigungsflüssigkeiten haben in der Regel eine definierte Zusammensetzung, die sie für bestimmte Zwecke besonders geeignet macht. Die Zusammensetzung bedingt zugleich auch die Begrenzungen in der Anwendung, so daß beispielsweise wäßrige Reinigungsflüssigkeiten in der Regel bei Temperaturen deutlich unter dem Gefrierpunkt nicht mehr eingesetzt werden können. Desgleichen verbietet sich der Einsatz leichtflüchtiger Lösungsmittel in tropischen Gegenden. Aufgrund dieser Beschränkungen tritt häufig der Fall ein, daß in einer Notfallsituation, beispielsweise bei einem Unfall, bei der Verschmutzung eines wichtigen Teils einer Anlage oder bei der Kontamination mit übelriechenden, verschmutzenden, giftigen oder ätzenden Substanzen, kein geeignetes Reinigungsmittel zur Verfügung steht. Hier besteht Bedarf nach einem universell einsetzbaren Reinigungsmittel für solche Fälle, das beispielsweise in einem Set oder einer Aufreißpackung ständig bereitsteht und bei Bedarf unmittelbar eingesetzt werden kann. Ein solches Reinigungsmittel sollte sowohl in Verbindung mit Wasser als auch wasserfrei einsetzbar sein und nach Verwendung problemlos mit dem Abwasser oder Hausmüll entsorgt werden können.

Diese Aufgabe wird erfindungsgemäß mit einer Reinigungsflüssigkeit gelöst, die als Komponenten (A) einen Polyalkylenglykolmonoalkylether und (B) ein Polyalkylenglykolfettsäurepartialglyzerid, und wahlweise wenigstens ein nicht-ionogenes flüssiges oberflächenaktives Mittel enthält.

Bevorzugte Zusammensetzungen sind in den Unteransprüchen näher definiert.

Die erfindungsgemäße Reinigungsflüssigkeit ist insbesondere für den Einsatz bei Notfällen geeignet. Beispielsweise findet sie Verwendung als Mittel zum Reinigen und/oder Dekontaminieren von Haut bei Verletzungen und/oder Kontaminationen bei Mensch und Tier. Sie kann aber gleichfalls zur Reinigung und/oder Dekontaminierung von Oberflächen, etwa von Maschinen, Scheiben, Möbeln, Pflanzen, etc. eingesetzt werden, ohne daß besondere Beschränkungen bestehen.

Die erfindungsgemäße Reinigungsflüssigkeit kann zwar mit Wasser angewandt bzw. gemischt werden, sie ist jedoch vorzugsweise ohne Wasser bzw. wasserunabhängig anwendbar, bei durchaus vergleichbarer schmutzbeseitigender, reinigender Wirkung. Sie kann dazu verwandt werden, Kontaminationen mit Chemikalien zu entfernen, wobei sie auch eine gewisse entgiftende Wirkung entfaltet. Sie ist weitgehend ungiftig, feuerungefährlich und wenig flüchtig und bleibt bei niedrigen Temperaturen weit unterhalb des Gefrierpunkts wie auch bei tropischer Hitze voll einsatzfähig. Als reinigendes Mittel für Oberflächen ersetzt sie übliche Reiniger wie Benzin, Aromaten, Terpentin, Alkohol, Chlorkohlenwasserstoffe oder Ether und Ester.

Bei den zum Einsatz kommenden Komponenten A bis D handelt es sich um handelsübliche Substanzen, die für verschiedene Zwecke auf dem Markt angeboten werden. Die Komponenten C und D können als Tenside oder oberflächenaktive Mittel bezeichnet werden, die lipophile und hydrophile Eigenschaften bei Wasserlöslichkeit oder -verträglichkeit aufweisen. Anstelle der Komponenten C und D können aber auch übliche nicht-ionogene oberflächenaktive Mittel eingesetzt werden, die allein oder in Kombination lipophile und hydrophile Eigenschaften haben. Natürlich können die Komponenten C und D auch in Verbindung mit weiteren nicht-ionogenen oberflächenaktiven Mitteln eingesetzt werden. Die nicht-ionogenen oberflächenaktiven Mittel sowohl herkömmlicher Struktur als auch der Strukturformeln C und D können sowohl wasserlöslich als auch wasserunlöslich sein. Wichtig ist jedoch, daß dabei die lipophilen und hydrophilen Eigenschaften gegeneinander ausbalanciert sind, um die erfindungsgemäße Reinigungsflüssigkeit mit dem notwendigen Anwendungsspektrum zu versehen.

Der als Komponente A verwandte Polyoxyalkylenglykolmonoalkylether ist vorzugsweise ein Mischblokkoligomeres oder -polymeres aus Propylenoxid und Ethylenoxid mit vorzugsweise bis zu 80 Oxyalkyleneinheiten und bis zu 6 Kohlenstoffatomen in der Ethereinheit. Besonders bevorzugt ist ein Polyoxyethylenpolyoxypropylenmonoalkylether mit einer der nachstehenden Formeln A

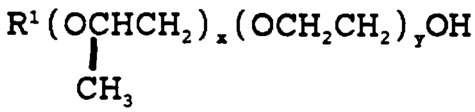 A 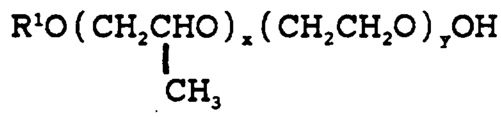

worin $R^1$ ein $C_1$-$C_6$-Alkylrest ist, x 2 bis 33 ist und y 3 bis 45 ist. Als Alkylrest kommt insbesondere der Butylrest in Frage; es können aber auch andere geradkettige oder Verzweigte Alkylreste mit bis zu 6 Kohlenstoffatomen verwandt werden. x hat dabei insbesondere einen Wert von 9 bis 20 und y von 10 bis 30.

Als Komponente B kommt insbesondere ein Polyalkylenglykolmonofettsäureglyzerid einer $C_6$-$C_{18}$-Fettsäure in Frage, insbesondere ein solches mit der nachstehenden Formel B

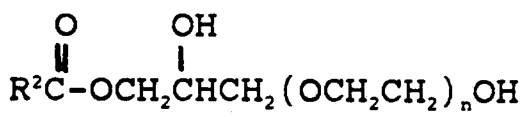 B

worin $R^2$ ein $C_5$-$C_{17}$-Alkyl oder -Alkenyl ist und n 2 bis 10 ist. Vorzugsweise enthalt das Glyzerid im Mittel 6 Ethylenoxideinheiten und ist $R^2$ ein $C_7$- und/oder $C_9$-Alkylrest. Die Komponente B kann auch ein polyoxethyliertes Fettsäurepartialglyzerid eine $C_6$-$C_{18}$-Fettsäure sein.

Das Polyalkylenglykolfettsäurepartialglyzerid der Komponente B kann selbst als nicht-ionogenes Tensid oder oberflächenaktives Mittel angesehen werden, ist aber in erster Linie als reinigende bzw. lösende Komponente anzusehen.

Neben den Komponenten A und B enthält die erfindungsgemäße Reinigungsflüssigkeit wahlweise ein nichtionogenes flüssiges oberflächenaktives Mittel, das zugleich lipophil und hydrophil ist. Hierbei kommt es darauf an, daß diese Eigenschaften gegeneinander ausbalanciert sind, was mit einem sowohl lipophil als auch hydrophil eingestellten Tensid erreicht werden kann, als auch durch eine Kombination eines lipophilen mit einem hydrophilen Tensid.

Als nicht-ionogenes flüssiges oberflächenaktives Mittel kann ein Polyoxyalkylensorbitol- und/oder Polyoxyalkylensorbitanmonofettsäureester verwandt werden. Letzteres hat insbesondere die nachfolgende Struktur C

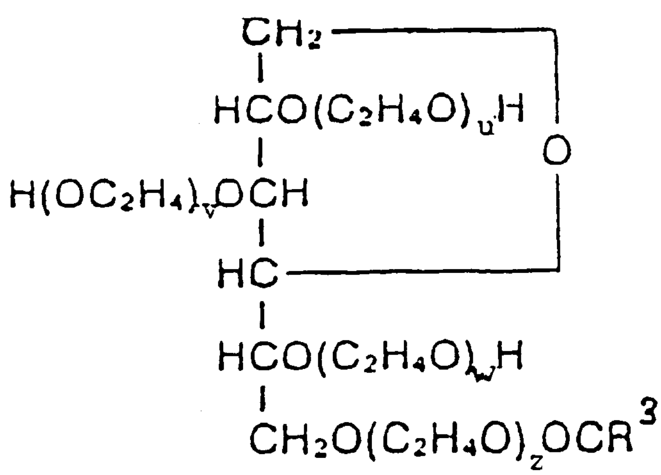 C

worin $R^3$ ein $C_7$-$C_{21}$-Alkyl- oder Alkenylrest ist und die Summe u + v + w + z einen Durchschnittswert von 10 bis 30 ergibt. Besonders bevorzugt sind Oleate mit einem Durchschnittswert der Summe von u + v + w + z von etwa 20. Der Sorbitanester C kann in Mischung mit dem entsprechenden Sorbitester vorliegen, bei dem dann die weiteren Hydroxylfunktionen ebenfalls oxyalkyliert sein können.

Alternativ oder zusätzlich kann die Reinigungsflüssigkeit als nicht-ionogenes flüssiges oberflächenaktives Mittel einen Polyoxyalkylenfettalkoholether enthalten, insbesondere einen solchen der nachstehenden Formel D

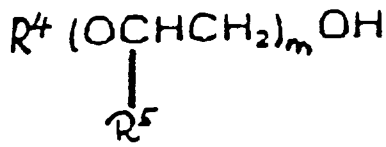

D

worin $R^4$ ein $C_6$-$C_{20}$-Alkylrest ist, $R^5$ H und/oder $CH_3$ und m einen Durchschnittswert von 4 bis 50 aufweist. Dabei ist $R^4$ besonders bevorzugt Stearyl, hat m einen Durchschnittswert von etwa 15, und ist $R^5$ $CH_3$ . Komponente D kann aber auch als Mischung der Polyoxyethylen- und -propylenverbindungen vorliegen oder als Mischoligomeres oder -polymeres.

In der erfindungsgemäßen Reinigungsflüssigkeit liegen die Komponenten A und B, wenn sie zusammen eingesetzt werden, vorzugsweise in einem Volumenverhältnis von 1:1 bis 4:1, insbesondere von etwa 2:1 vor. Die Komponente C kann in einer Menge von bis zu 40 Vol.-%, vorzugsweise etwa 20 Vol.-% zugegen sein. Komponente D liegt zweckmäßigerweise in einem volumenanteil von bis zu 20% vor, vorzugsweise von etwa 6%. Das Volumenverhältnis A/B kann auch 1:1 bis 1:4 sein.

Bevorzugte Zusammensetzungen der erfindungsgemäßen Reinigungsflüssigkeit enthalten die Komponenten A bis D in den folgenden Volumenanteilen:
A 40 bis 60%; B 20 bis 30%; C 15 bis 25% und D 2 bis 10%. Dabei hat sich die nachstehende Zusammensetzung als besonders zweckmäßig erwiesen:

| | |
|---|---|
| A Polyoxyethylenpolyoxypropylenmonobutylether | 50% |
| B Polyoxyethylen-Oktan/Dekansäureglyzeride | 24% |
| C Polyoxyethylensorbitanmonooleat | 20% |
| D Polyoxypropylenstearylether | 6%. |

Die erfindungsgemäße Reinigungsflüssigkeit kann übliche Zusatzstoffe, wie Stabilisatoren, Farbstoffe, Konservierungsmittel, Desinfektionsmittel, Geruchsstoffe, etc., je nach Bedarf, enthalten. In manchen Fällen ist es zweckmäßig, sie als Reinigungsgel einzustellen, in welchem Fall sie übliche Verdickungsmittel enthält, sofern die natürliche Viskosität noch nicht ausreicht. Da die erfindungsgemäße Reinigungsflüssigkeit beispielsweise auch als Gel vorliegen kann, wird sie bevorzugt als Reinigungszusammensetzung bezeichnet.

Die erfindungsgemäße Reinigungszusammensetzung kann auch ohne weitere Zusätze an sich desinfizierend wirken; sie kann deshalb auch als Desinfektionsmittel bei Mensch und Tier und bei Gegenständen verwendet werden. Obwohl es nicht notwendig ist, weitere Zusätze zu verwenden, können auch dem Fachmann bekannte Zusätze, beispielsweise auch andere Desinfektionsmittel, beigemischt werden. Die erfindungsgemäße Reinigungszusammensetzung kann auch als Grundlage für Desinfektionsmittel (als Vehiculum und Adjuvans) verwendet werden.

Die erfindungsgemäße Reinigungszusammensetzung findet beim dekontaminieren von Mensch und Tier und von Gegenständen Verwendung, beispielsweise zur Entfernung von Giften.

Die erfindungsgemäße Reinigungszusammensetzung ist auch als Mittel gegen Hauterkrankungen, beispielsweise von Parakeratosen, Hyperkeratosen, Seborrhoe, Ichthyosis, Akne, Ekzemen und anderen Hauterkrankungen, allgemein also zur medizinischen Hautbehandlung und Hautreinigung bei Mensch und Tier einsetzbar.

**Patentansprüche**

**1.** Reinigungsflüssigkeit, insbesondere für Notfallsets, welche als Bestandteile
(A) einen Polyalkylenglykolmonoalkylether und
(B) ein Polyalkylenglykolfettsäurepartialglyzerid, und wahlweise wenigstens ein nicht-ionogenes flüssiges oberflächenaktives Mittel enthält.

**2.** Reinigungsflüssigkeit nach Anspruch 1, dadurch gekennzeichnet, daß Komponente (A) ein Polyoxyethylenpolyoxypropylenmonoalkylether mit bis zu 80 Oxyalkyleneinheiten und bis zu 6 Kohlenstoffatomen in der Ethereinheit ist.

**3.** Reinigungsflüssigkeit nach Anspruch 2, dadurch gekennzeichnet, daß der Polyoxyethylenpolyoxypropylenmonoalkylether eine der nachfolgenden Formeln A aufweist

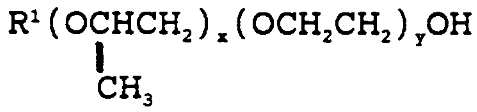

A

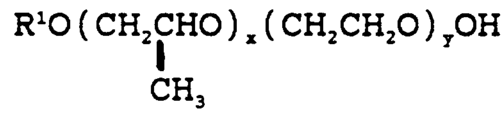

worin $R^1$ ein $C_1$-$C_6$-Alkylrest ist, x 2 bis 33 ist und y 3 bis 45 ist.

**4.** Reinigungsflüssigkeit nach Anspruch 3, dadurch gekennzeichnet, daß in Formel A $R^1$ ein Butylrest ist, x 9 bis 20 ist und y 10 bis 30 ist.

**5.** Reinigungsflüssigkeit nach einem der Ansprüche 1 bis 4, dadurch gekennzeichnet, daß die Komponente B ein Polyalkylenglykolmonofettsäureglyzerid einer $C_6$-$C_{18}$-Fettsäure oder ein polyoxethyliertes Fettsäurepartialglyzerid einer $C_6$-$C_{18}$-Fettsäure ist.

**6.** Reinigungsflüssigkeit nach Anspruch 5, dadurch gekennzeichnet, daß das Polyalkylenglykol-monofettsäureglyzerid die Formel B hat

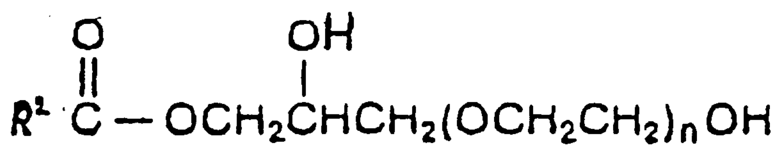

B

worin $R^2$ ein $C_5$-$C_{17}$-Alkyl oder -Alkenyl ist und n 2 bis 10 ist und vorzugsweise einen Durchschnittswert von 6 hat.

**7.** Reinigungsflüssigkeit nach Anspruch 6, dadurch gekennzeichnet, daß in Formel B $R^2$ ein $C_7$- und/oder $C_9$-Alkylrest ist und n einen mittleren Wert von 6 hat.

**8.** Reinigungsflüssigkeit nach einem der Ansprüche 1 bis 7, dadurch gekennzeichnet, daß der Zusatz an nichtionogenem flüssigem oberflächenaktivem Mittel zugleich lipophil und hydrophil ist.

**9.** Reinigungsflüssigkeit nach einem der Ansprüche 1 bis 8, dadurch gekennzeichnet, daß das nichtionogene flüssige oberflächenaktive Mittel ein Polyoxyalkylensorbitmonofettsäureester und/oder Polyoxyalkylensorbitanmonofettsäureester ist (Komponente C).

**10.** Reinigungsflüssigkeit nach Anspruch 9, dadurch gekennzeichnet, daß der Polyoxyalkylensorbitanmonofettsäureester die Formel C hat

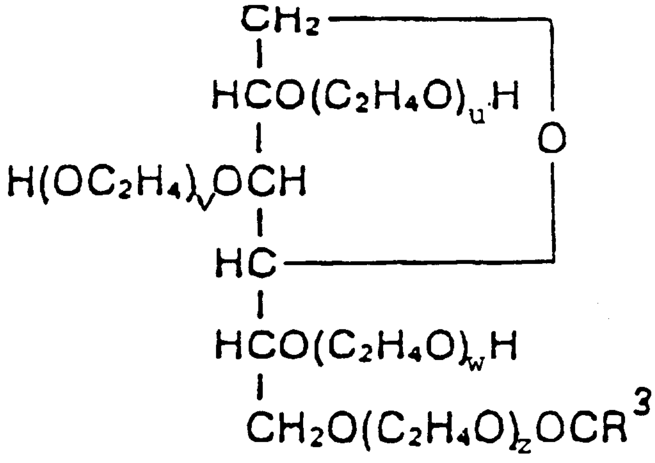

C

worin $R^3$ ein $C_7$-$C_{21}$ Alkyl oder Alkenylrest ist und die Summe u + v + w + z einen Durchschnittswert von 10 bis 30 ergibt.

**11.** Reinigungsflüssigkeit nach Anspruch 10, dadurch gekennzeichnet, daß der Ester der Formel C ein Oleat mit einem Durchschnittswert der Summe von u + v + w + z von 20 ist.

**12.** Reinigungsflüssigkeit nach einem der Ansprüche 1 bis 11, dadurch gekennzeichnet, daß sie als nichtionogenes flüssiges oberflächenaktives Mittel einen Polyoxyalkylenfettalkoholether enthält (Komponente D).

**13.** Reinigungsflüssigkeit nach Anspruch 12, dadurch gekennzeichnet, daß der Polyoxyalkylenfettalkoholether der Formel D entspricht

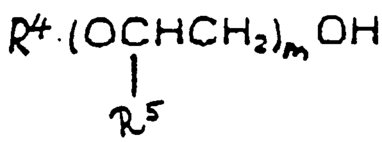

D

worin $R^4$ ein $C_6$-$C_{20}$-Alkylrest ist, $R^5$ H und/oder $CH_3$ ist und m einen Durchschnittswert von 4 bis 50 aufweist.

**14.** Reinigungsflüssigkeit nach Anspruch 13, dadurch gekennzeichnet, daß $R^4$ Stearyl ist und m einen Durchschnittswert von 15 hat.

**15.** Reinigungsflüssigkeit nach einem der Ansprüche 1 bis 14, dadurch gekennzeichnet, daß das Volumenverhältnis A/B 1:1 bis 4:1 oder A/B 1:1 bis 1:4 ist.

**16.** Reinigungsflüssigkeit nach Anspruch 15, dadurch gekennzeichnet, daß das Verhältnis A/B etwa 2:1 ist.

**17.** Reinigungsflüssigkeit nach einem der Ansprüche 1 bis 16, dadurch gekennzeichnet, daß Komponente C in einer Menge von bis zu 40 Vol.-%, vorzugsweise etwa 20 Vol.-% zugegen ist.

**18.** Reinigungsflüssigkeit nach einem der Ansprüche 1 bis 17, dadurch gekennzeichnet, daß die Komponente D in einem volumenanteil von bis zu 20%, vorzugsweise etwa 6%, zugegen ist.

**19.** Reinigungsflüssigkeit nach einem der Ansprüche 1 bis 18, gekennzeichnet durch die folgenden Volumenanteile der Komponenten A bis D:

| | |
|---|---|
| A | 40 bis 60% |
| B | 20 bis 30% |
| C | 15 bis 25% |
| D | 2 bis 10%. |

**20.** Reinigung flüssigkeit nach Anspruch 19 mit den folgenden Volumenanteilen der Komponenten A bis D:

| | |
|---|---|
| A | 50% |
| B | 24% |
| C | 20% |
| D | 6%. |

**21.** Reinigungsflüssigkeit nach einem der vorstehenden Ansprüche, dadurch gekennzeichnet, daß sie übliche Zusatzstoffe, wie Stabilisatoren, Farbstoffe, Konservierungsmittel, Desinfektionsmittel, Geruchsstoffe, etc. enthält.

**22.** Reinigungsflüssigkeit nach einem der vorstehenden Ansprüche, dadurch gekennzeichnet, daß sie viskos als Gel eingestellt ist.

**23.** Verwendung der Reinigungsflüssigkeit nach einem oder mehreren der vorstehenden Ansprüche zur Herstellung eines Arzneimittels zur medizinischen Hautbehandlung und Hautreinigung.

**24.** Verwendung der Reinigungsflüssigkeit nach einem oder mehreren der vorstehenden Ansprüche zur Herstellung eines Mittels zum Reinigen und/oder Dekontaminieren von Haut bei Verletzungen und/oder Kontaminationen bei Mensch und Tier.

**25.** Verwendung der Reinigungsflüssigkeit nach einem oder mehreren der vorstehenden Ansprüche zur Herstellung eines Mittels zum Reinigen und/oder Dekontaminieren von verunreinigten Oberflächen.

**26.** Verwendung der Reinigungsflüssigkeit nach einem oder mehreren der vorstehenden Ansprüche zur Herstellung eines Desinfektionsmittels und/oder als Grundlage für ein Desinfektionsmittel.

## Claims

**1.** A cleaning liquid, particularly for use in emergency sets, which contains as components
(A) a polyalkyleneglycol monoalkyl ether and
(B) a polyalkyleneglycol fatty acid partial glyceride and optionally at least one non-ionogenic liquid surface-active agent.

**2.** A cleaning liquid according to claim 1, characterized in that the component (A) is a polyoxyethylenepolyoxypropylene monoalkyl ether having up to 80 oxyalkylene units and up to 6 carbon atoms in the ether unit.

**3.** A cleaning liquid according to claim 2, characterized in that the polyoxyethylene-polyoxypropylene monoalkyl ether has one of the following formulas A

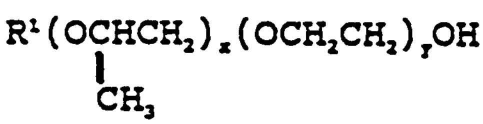

A

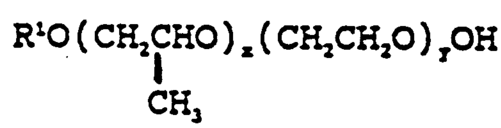

wherein $R^1$ is an alkyl residue having 1 to 6 carbon atoms, x equals 2 to 33, and y equals 3 to 45.

**4.** A cleaning liquid according to claim 3, characterized in that in formula A $R^1$ is a butyl residue, x equals 9 to 20, and y equals 10 to 30.

**5.** A cleaning liquid according to any of claims 1 to 4, characterized in that component B is a polyalkyleneglycol monofatty acid glyceride of a fatty acid having 6 to 18 carbon atoms or a polyoxyethylated fatty acid partial glyceride of a fatty acid having 6 to 18 carbon atoms.

**6.** A cleaning liquid according to claim 5, characterized in that the polyalkyleneglycol monofatty acid glyceride has the formula B

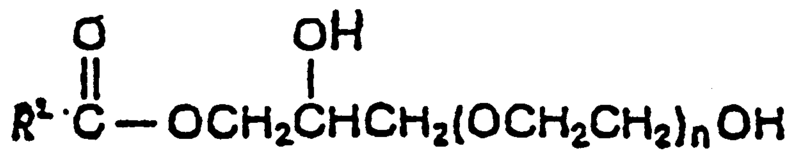

B

wherein $R^2$ is an alkyl or alkenyl having 5 to 17 carbon atoms and n equals 2 to 10 and preferably has an average value of 6.

**7.** A cleaning liquid according to claim 6, characterized in that in formula B $R^2$ is an alkyl residue having 7 and/or 9 carbon atoms and n has an average value of 6.

**8.** A cleaning liquid according to any of claims 1 to 7, characterized in that the additive consisting of a non-ionogenic liquid surface-active agent is both lipophilic and hydrophilic.

**9.** A cleaning liquid according to any of claims 1 to 8, characterized in that the non-ionogenic liquid surface-active agent is a polyoxyalkylenesorbitol monofatty acid ester and/or a polyoxyalkylenesorbitan monofatty acid ester (component C).

**10.** A cleaning liquid according to claim 9, characterized in that the polyoxyalkylenesorbitan montofatty acid ester has the formula C

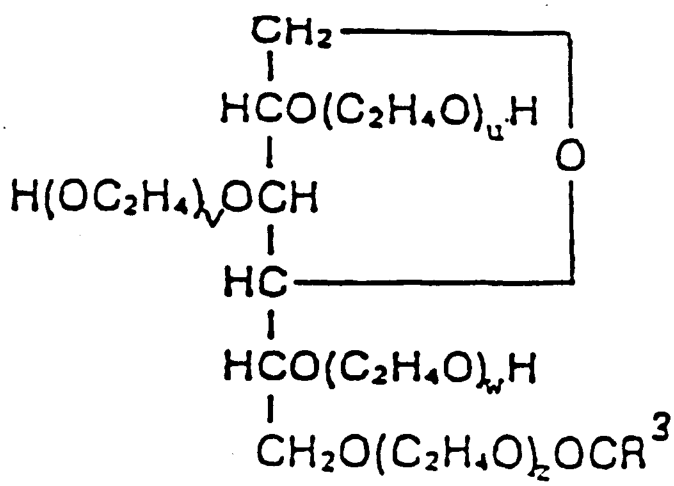

C

wherein $R^3$ is an alkyl residue or alkenyl residue having 7 to 21 carbon atoms and the sum u + v + w + z has an average value of 10 to 30.

**11.** A cleaning liquid according to claim 10, characterized in that the ester of formula C is an oleate in which the sum of u + v + w + z has an average value of 20.

**12.** A cleaning liquid according to any of claims 1 to 11, characterized in that it contains a polyoxyalkylene fatty alcohol ether (component D) as a non-ionogenic liquid surface-active agent.

**13.** A cleaning liquid according to claim 12, characterized in that the polyoxyalkylene fatty alcohol ether has the formula D

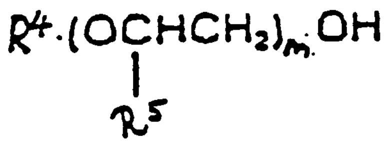

D

wherein $R^4$ is an alkyl residue having 6 to 20 carbon atoms, $R^5$ is H and/or $CH_3$, and m has an average value of 4 to 50.

**14.** A cleaning liquid according to claim 13, characterized in that $R^4$ is stearyl and m has an average value of 15.

**15.** A cleaning liquid according to any of claims 1 to 14, characterized in that the volume ratio A/B is 1:1 to 4:1 or 1:1 to 1:4.

**16.** A cleaning liquid according to claim 15, characterized in that the ratio A/B is about 2:1.

**17.** A cleaning liquid according to any of claims 1 to 16, characterized in that component C is present in an amount of up to 40% by volume, preferably of about 20% by volume.

**18.** A cleaning liquid according to any of claims 1 to 17, characterized in that component D is present in an amount of up to 20% by volume, preferably of about 6% by volume.

**19.** A cleaning liquid according to any of claims 1 to 18, characterized by the following contents of components A to D by volume:

| | |
|---|---|
| A | 40 to 60% |
| B | 20 to 30% |
| C | 15 to 25% |
| D | 2 to 10%. |

**20.** A cleaning liquid according to claim 19 with the following contents of components A to D by volume:

| | |
|---|---|
| A | 50% |
| B | 24% |
| C | 20% |
| D | 6% |

**21.** A cleaning liquid according to any of the preceding claims, characterized in that it contains conventional additives, such as stabilizers, dyestuffs, preservatives, disinfectants, odorous substances, etc.

**22.** A cleaning liquid according to any of the preceding claims, characterized in that it is prepared as a viscous gel.

**23.** The use of the cleaning liquid according to any of the preceding claims for the preparation of a medicament for the treatment and cleaning of skin.

**24.** The use of the cleaning liquid according to any of the preceding claims for the preparation of an agent for cleaning and/or decontaminating skin in the case of injuries and/or a decontamination of human beings and animals.

**25.** The use of the cleaning liquid according to any of the preceding claims for the preparation of an agent for cleaning and/or decontaminating soiled surfaces.

**26.** The use of the cleaning liquid according to any of the preceding claims for the preparation of a disinfectant and/or as a basis for a disinfectant.

**Revendications**

**1.** Solution de nettoyage plus particulièrement destinée à faire partie d'une trousse de secours contenant à titre de constituant (A) un éther monoalkylique de polyalkylèneglycol et (B) un ester partiel de polyalkylèneglycol avec un ou plusieurs acides gras et éventuellement au moins un agent tensioactif liquide non-ionogène.

**2.** solution de nettoyage selon la revendication 1, caractérisée en ce que le constituant (A) est en éther monoalkylique de polyoxyéthylène-polyoxypropylène ayant jusqu'à 80 unités oxyalkylène et jusqu'à 6 atomes de carbone dans la partie éther.

**3.** Solution de nettoyage selon la revendication 2 caractérisée en ce que l'éther monoalkylique de polyoxyéthylène-polyoxypropylène a l'une des formules A suivantes:

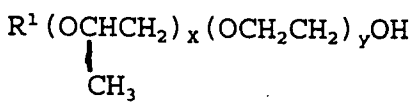

A

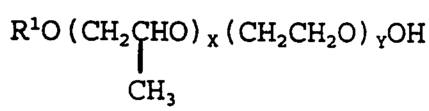

dans laquelle $R^1$ est un radical alkyle présentant de 1 à 6 atomes de carbone, X est de 2 à 33 et Y est de 3 à 45.

**4.** Solution de nettoyage selon la revendication 3 caractérisée en ce que dans la formule A, $R^1$ est un radical butyle, X est de 9 à 20 et Y est de 10 à 30.

**5.** Solution de nettoyage selon l'une quelconque des revendications 1 à 4 caractérisée en ce que le constituant B est un monoester de polyalkylèneglycol avec un acide gras, ledit acide gras présentant de 6 à 18 atomes de carbone ou un ester partiel de glycérol polyoxyethylé avec un acide gras présentant de 6 à 18 atomes de carbone.

**6.** Solution de nettoyage selon la revendication 5 caractérisée en ce que le monoester de polyalkylèneglycol avec un acide gras a la formule B :

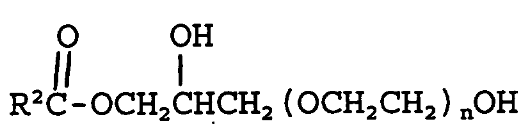

B

Dans laquelle $R^2$ est un radical alkyle ou alcényle présentant de 5 à 17 atomes de carbones et n vaut 2 à 10 et de préférence n a une valeur moyenne de 6.

**7.** Solution de nettoyage selon la revendication 6 caractérisée en ce que dans la formule B, $R^2$ est un radical alkyle ayant 7 et/ou 9 atomes de carbone et n a une valeur moyenne de 6.

**8.** Solution de nettoyage selon l'une quelconque des revendications 1 à 7 caractérisée en ce que l'additif constitué de l'agent tensioactif liquide non-ionogène est à la fois lipophile et hydrophile.

**9.** Solution de nettoyage selon l'une quelconque des revendications 1 à 8 caractérisée en ce que l'agent tensioactif liquide non-ionogène est un monoester de polyoxyalkylènesorbitol avec un acide gras et/ou un monoester d'un polyoxyalkylènesorbitanne avec un acide gras (constituant C).

**10.** Solution de nettoyage selon la revendication 9 caractérisée en ce que le monoester de polyoxyalkylènesorbitanne avec un acide gras a la formule C suivante:

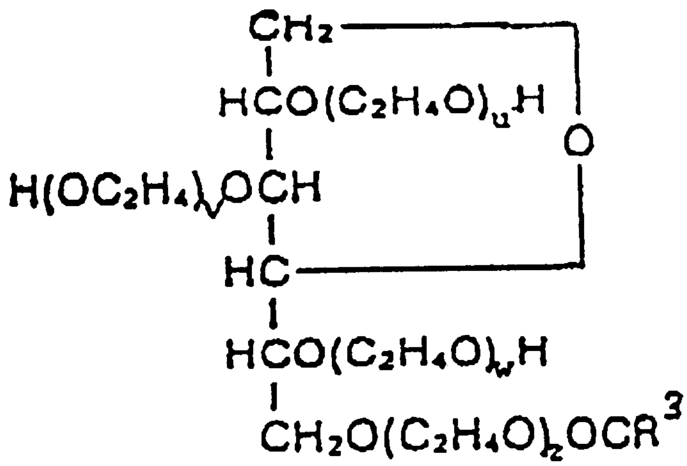

C

dans laquelle $R^3$ est un radical alkyle ou alcényle ayant de 7 à 21 atomes de carbone et la somme u + v + w + z a une valeur moyenne comprise entre 10 et 30.

**11.** Solution de nettoyage selon la revendication 10 caractérisée en ce que l'ester de formule C est un oléate dans lequel la somme u + v + w + z a une valeur moyenne de 20.

**12.** Solution de nettoyage selon l'une quelconque des revendications 1 à 11 caractérisée en ce qu'elle contient un éther de polyoxyalkylène avec un alcool gras (constituant D) en tant qu'agent tensioactif liquide non-ionogène.

**13.** Solution de nettoyage selon la revendication 12 caractérisée en ce que l'éther de polyoxyalkylène avec un alcool gras a la formule D suivante:

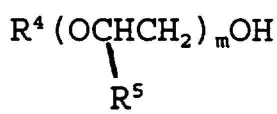

D

dans laquelle $R^4$ est un radical alkyle présentant de 6 à 20 atomes de carbone $R^5$ est H et/ou $CH_3$ et m a une valeur moyenne de 4 à 50.

**14.** Solution de nettoyage selon la revendication 13 caractérisée en ce que $R^4$ est stéaryle et m a une valeur moyenne de 15.

**15.** Solution de nettoyage selon l'une quelconque des revendications 1 à 14 caractérisée en ce que le rapport volumique A/B est compris entre 1:1 et 4:1 ou 1:1 et 1:4.

**16.** Solution de nettoyage selon la revendication 15 caractérisée en ce que le rapport A/B est d'environ 2:1.

**17.** Solution de nettoyage selon l'une quelconque des revendications 1 à 16 caractérisée en ce que le constituant C est présent en une quantité représentant jusqu'à 40% en volume, de préférence environ 20% en volume.

**18.** Solution de nettoyage selon l'une quelconque des revendications 1 à 17 caractérisée en ce que le constituant D est présent en une quantité représentant jusqu'à 20% en volume, de préférence environ 6% en volume.

**19.** Solution de nettoyage selon l'une quelconque des revendications 1 à 18 caractérisée par les teneurs suivantes en constituants A à D, en volume:

| | |
|---|---|
| A | 40 à 60 % |
| B | 20 à 30 % |
| C | 15 à 25 % |
| D | 2 à 10 % |

**20.** Solution de nettoyage selon la revendication 19 présentant les teneurs suivantes en constituants A à D, en volume:

| | |
|---|---|
| A | 50 % |
| B | 24 % |
| C | 20 % |
| D | 6 % |

**21.** Solution de nettoyage selon l'une quelconque des revendications précédentes caractérisée en ce qu'elle contient des additifs conventionnels, tels que des stabilisants, des colorants, des conservateurs, des désinfectants, des substances odorantes, etc...

**22.** Solution de nettoyage selon l'une quelconque des revendications précédentes caractérisée en ce qu'elle est préparée sous la forme d'un gel visqueux.

**23.** Utilisation d'une solution de nettoyage selon l'une quelconque des revendications précédentes pour la préparation d'un médicament approprié à des soins médicaux et au nettoyage de la peau.

**24.** Utilisation d'une solution de nettoyage selon l'une quelconque des revendications précédentes pour la préparation d'un agent destiné au nettoyage et/ou à la désinfection de la peau dans le cas de blessures et/ou à la désinfection des hommes et des animaux.

**25.** Utilisation d'une solution de nettoyage selon l'une quelconque des revendications précédentes pour la préparation d'un agent destiné au nettoyage et/ou à la désinfection de surfaces souillées.

**26.** Utilisation d'une solution de nettoyage selon l'une quelconque des revendications précédentes pour la préparation d'un désinfectant et/ou en tant que base pour un désinfectant.